# EUROPEAN PATENT APPLICATION

(11) **EP 2 947 587 A1**
(43) Date of publication of application: **25.11.2015**
(21) Application number: 14191475.4
(22) Date of filing: 03.11.2014
(51) Int. Cl.: G06F 19/00

(54) **Methods and systems for processing sporting performance data**

(30) Priority: 21.05.2014 AU 2014901899
(71) Applicant: Rip Curl International Pty Ltd, Torquay, Victoria 3228 (AU)
(72) Inventor: Helm, Shane, Torquay, Victoria 3228 (AU)
(74) Representative: V.O.

(57) **Abstract**

Described herein is a computer processing system 100 including a processing unit 106. The processing unit is configured to access activity data relating to a user's movements during a session. The processing unit processes said activity data to identify one or more wave start points 206 and one or more wave end points 210; and estimate one or more waves 202 caught by the user during the session, each wave being defined by a wave start point and a corresponding wave end point.

## Description

### Field of the invention

This invention relates generally to methods and systems for processing sporting performance data.

### Background of the invention

Systems for monitoring, recording, processing, and analysing sporting performance exist. Such systems typically include a portable device for monitoring and recording data relating to various aspects of an athlete's performance and software (run either on the portable device itself or an alternative computer) to assist in analysing and/or visualising that data.

For example, systems for monitoring and analysing the activity of a cyclist during a ride exist. Such systems can include a portable bike computer (carried on a bike) which monitors and records, for example, Global Positioning System (GPS) data, speed data, cadence data, heart rate data, temperature data, power data. The data collected by the portable bike computer can then be transferred to another computer device to be processed for analysis and presentation.

Similar systems for monitoring and analysing the activity of joggers/runners exist and use similar principles.

Such systems are not, however, suitable for use with all sports/activities.

It would be desirable to provide methods and systems which can be used to record, process, analyse and/or visualise data for alternative activities. In addition, or in the alternative, it would be desirable to provide a useful alternative to existing methods and systems for monitoring, recording, and/or analysing athletic performance.

Reference to any prior art in this specification is not, and should not be taken as, an acknowledgment or any form of suggestion that this prior art forms part of the common general knowledge in Australia or any other jurisdiction or that this prior art could reasonably be expected to be ascertained, understood and regarded as relevant by a person skilled in the art.

### Summary of the invention

Described herein is a computer processing system including: a processing unit configured to: access activity data relating to a user's movements during a session; process said activity data to identify one or more wave start points and one or more wave end points; and estimate one or more waves caught by the user during the session, each wave being defined by a wave start point and a corresponding wave end point.

The activity data may include speed data recorded over the course of the session; and the processing unit may be configured to process said speed data to identify one or more wave start and/or wave end points.

Identifying a wave start point may include processing the activity data to identify a point in the activity data where speed exceeds a predetermined on-wave speed threshold.

Identifying a wave start point may include processing the activity data to identify a point in the activity data where speed has exceeded a predetermined on-wave speed threshold for longer than a predetermined on-wave time window.

Identifying a wave end point may include processing the activity data to identify a point in the activity data where speed is less than a predetermined off-wave speed threshold.

Identifying a wave end point may include processing the activity data to identify a point in the activity data where speed has been less than a predetermined off-wave speed threshold for longer than a predetermined off-wave time window.

The activity data may include positional data recorded over the course of the session; and the processing unit may be configured to process said positional data to identify one or more wave start and/or a wave end points.

Identifying a wave start point may include processing the activity data to identify a point in the activity data where position deviates by more than a predetermined on-wave angle.

Identifying a wave end point may include processing the activity data to identify a point in the activity data where position deviates by more than a predetermined off-wave angle.

The processing unit may be further configured to calculate a total on-wave time during the session, the total on-wave time being the sum of the durations of the one or more waves caught.

The computer processing system may further include a display; the activity data may include positional data recorded over the course of the session; and the processing unit may be further configured to display a position of at least one of the waves estimated to have been caught during the session.

The processing unit may be configured to display positions of waves on a map or image of the location in which the session took place.

The computer processing system may further include non-transient memory, wherein the non-transient memory stores computer readable instructions, and wherein configuration of the processing unit is achieved by the execution of computer readable instructions by the processing unit.

The activity data may be stored on the non-transient memory.

The activity data may be recorded by a portable device carried by the user during the session, and wherein the activity data is received by the computer processing system from the portable device.

The computer processing system may further include a position sensor, and wherein: the activity data includes data sensed by the position sensor; and the computer processing system may be a portable device configured to be carried by the user during the session.

The portable device may be a wearable device.

Also described herein is a computer-implemented method for estimating one or more waves caught by a user during a session, the method including: accessing activity data relating to a user's movements during a session; operating a computer processing unit to process said activity data to identify one or more wave start points and one or more wave end points; and estimating one or more waves caught by the user during the session, each wave being defined by a wave start point and a corresponding wave end point.

The activity data may include speed data recorded over the course of the session; and the method may include identifying one or more wave start and/or wave end points from the speed data.

Identifying a wave start point may include identifying a point in the activity data where speed exceeds a predetermined on-wave speed threshold.

Identifying a wave start point may include identifying a point in the activity data where speed has exceeded a predetermined on-wave speed threshold for longer than a predetermined on-wave time window.

Identifying a wave end point may include identifying a point in the activity data where speed is less than a predetermined off-wave speed threshold.

Identifying a wave end point may include identifying a point in the activity data where speed has been less than a predetermined off-wave speed threshold for longer than a predetermined off-wave time window.

The activity data may include positional data recorded over the course of the session; and the method may include identifying one or more wave start and/or a wave end points from the positional data.

Identifying a wave start point may include identifying a point in the activity data where position deviates by more than a predetermined on-wave angle.

Identifying a wave end point may include identifying a point in the activity data where position deviates by more than a predetermined off-wave angle.

The computer-implemented method may further include calculating a total on-wave time during the session, the total on-wave time being the sum of the durations of the one or more waves caught.

The computer-implemented method may further include displaying a position of at least one of the waves estimated to have been caught during the session on a display.

Positions of waves estimated to have been caught may be displayed on a map or image of the location in which the session took place.

The activity data may include data generated by a position sensor.

Also described herein is a non-transient computer readable medium storing instructions executable by a computer processing unit to cause a computer processing system to perform a computer implemented method as described above.

Also described herein is a computer implemented method for processing activity data recorded by a portable device carried by a surfer during a surf session, the activity data being processed to identify one or more waves caught by the surfer during the surf session.

The portable device may include a GPS receiver, and the activity data may include GPS data.

The computer implemented method may include processing the GPS data according to one or more wave recognition criteria to determine the one or more waves caught by the surfer.

According to the wave recognition criteria one or more wave start points and one or more wave end points may be identified in the GPS data. A consecutive pair of identified wave start and wave end points may define a wave that has been caught by the surfer.

The wave recognition criteria may take the speed of the surfer into account to identify wave start and wave end points.

The wave recognition criteria may also, or alternatively, take changes in direction made by the surfer into account to identify wave start and wave end points.

The wave recognition criteria may also, or alternatively, take acceleration data into account to identify wave start and wave end points.

Acceleration data may be obtained from an accelerometer.

The computer-implemented method may be implemented by a processing unit carried by the portable device.

The portable device may be a wearable device.

The portable device may be a watch.

The portable device may be carried in a wetsuit pocket.

The computer-implemented method may be implemented by a processing unit of a computer processing system separate to the portable device, the computer processing system receiving activity data from the portable device.

The method may further include displaying information about the surf session. Such information may include one or more of the following: a number of waves caught during the surf session; a top speed reached during the surf session; a longest distance travelled on a wave during the surf session; a distance paddled during the surf session; a length of the surf session; a total distance travelled during the surf session; a visual indication of the location of one or more waves caught during the surf session.

The method may further include obtaining and recording or displaying additional data relevant to the surf session.

The additional data may include one or more of: map data obtained from a map data provider; satellite image data obtained from a satellite image data provider; surf condition data obtained from a surf condition data provider; weather data obtained from a weather data provider.

The method may further include sharing information about the surf session with one or more other people.

Also described herein is a non-transient computer readable medium storing instructions executable by a processing unit to cause a computer processing system to perform a computer implemented method as described above.

Also described herein is a computer processing system including a processing unit and memory, the memory storing instructions executable by the processing unit to perform a computer implemented method as described above.

The computer processing system may further include a GPS receiver for receiving GPS data and storing at least some of said GPS data to said memory.

### Brief description of accompanying drawings

Embodiments and features of the present invention will be described with reference to the accompanying figures in which:
Figure 1 is a block diagram of computer processing systems;
Figure 2A is a schematic example of detecting wave start and end points using a speed based criteria;
Figure 2B is a flowchart illustrating a method of analysing activity data to detect wave start points;
Figure 2C is a flowchart illustrating a method of analysing activity data to detect wave end points;
Figure 3A is a schematic example of detecting wave start and end points using an angular deviation based criteria;
Figure 3B is a flowchart illustrating another method of analysing activity data to detect wave start points;
Figure 3C is a flowchart illustrating another method of analysing activity data to detect wave end points;
Figure 4 is a schematic example of detecting wave start and end points using both speed and angular deviation based criteria;
Figure 5A illustrates schematically an example of lost track points due to GPS signal dropout;
Figure 5B illustrates schematically an example of erroneous track points; and
Figures 6A to 6D illustrate user interface screens for displaying surf session and user profile information.

### Description of embodiments

The present invention is related to methods and systems for use in processing sporting performance data.

In one embodiment the systems/methods described herein are particularly suitable for processing data relating to surfing (e.g. on a surfboard), and various features of the invention will be described in that context.

### Portable device

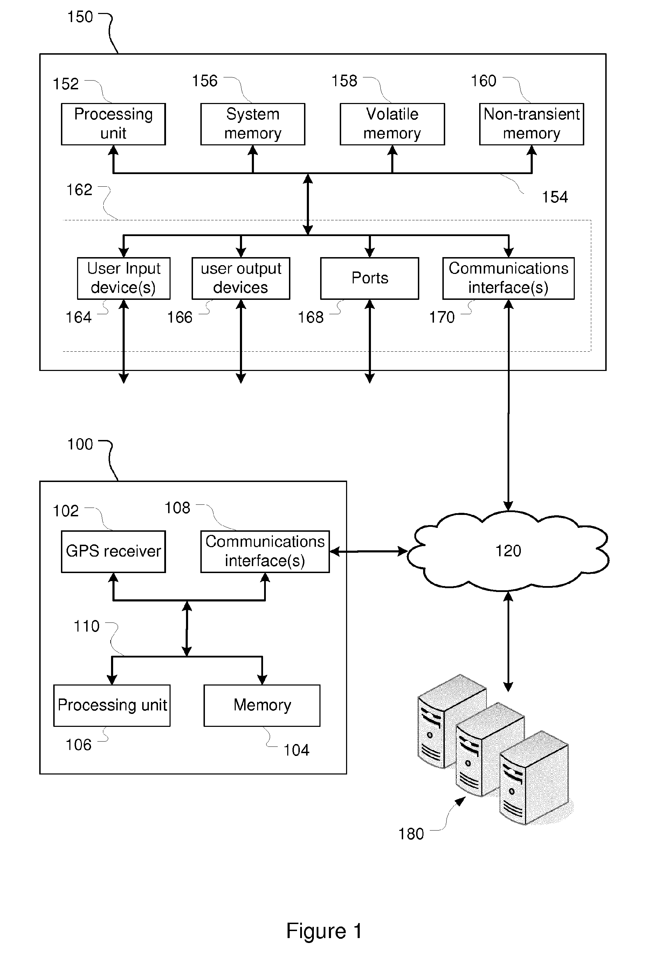

In order to collect data relating to the sport or activity (e.g. a surfing session) the activity participant (e.g. surfer) carries a portable device whilst undertaking the activity. Figure 1 includes a functional block diagram of one example of a portable device 100.

The portable device 100 may perform a variety of functions, but at the least will record activity data which includes positional information of the surfer over the course of the surfing session and allow for communication of this data to other computer processing devices or systems such as system 150.

To achieve this, the portable device 100 of the present embodiment includes: a GPS receiver 102 which receives GPS data; non-transient memory 104 for storing information recorded by the GPS receiver 102; a processing unit 106; and communications interface 108 operable to communicate data to (and receive data from) other computer processing systems such as system 150 (described further below). The communications interface 108 may provide for wired communication (e.g. using USB, FireWire, eSata, Thunderbolt, or other hardware and communications protocols) and/or wireless communication (e.g. using BlueTooth, infrared, WiFi, 3G, 4G, or other hardware and communications protocols). The various functions/components of the portable device 100 are interconnected by a communications bus 110.

Portable device 100 will also include a power supply (not shown) for powering the device 100, and one or more input/output devices (not shown) by which a user interacts with the portable device 100. The specific controls provided will depend on the functionality to be offered by device 100 itself. In one simple embodiment input may be limited to a control for turning the device on and off, and output limited to an interface for communicating data with another computer processing system. In other embodiments the device 100 may include multiple input/output components (e.g. provided by buttons and/or a touch screen display) to allow for additional functionality to be accessed on/performed by the portable device 100 itself.

The portable device 100 may also include additional components (not shown in Figure 1) for collecting additional activity data (to be stored on memory such as memory 104) and/or provide additional functionality. By way of non-limiting example, the portable device may further include one or more of the following: a temperature sensor for collecting temperature data over the course of the activity; a heart rate monitor (or a heart rate monitor receiver for receiving heart rate information from a separate heart rate monitor) for collecting heart rate data over the course of the activity; one or more accelerometers for collecting acceleration data over the course of the activity; buttons; a display (e.g. LCD, touch screen, or other); a speaker; a light.

The portable device 100 itself may take a variety of forms. For example, the portable device 100 may be a wearable device such as a watch, armband or similar. Alternatively, the portable device may be designed to be carried in a pocket or pouch, for example in a wetsuit pocket, a pair of board shorts or other garments, specially designed to securely hold the portable device 100. In another example, the portable device may be embedded in a wetsuit/swimwear, for example between two layers of a garment.

Given the portable device 100 is (in the surfing example) to be carried in the water it will advantageously be water resistant to a suitable level. Alternatively, the device may be intended to be housed in a water resistant pocket/pouch in which case water resistivity of the device itself may not be essential.

### Computer processing system

As noted, the portable device 100 is configured to communicate data with and receive data from other computer processing devices or systems such as system 150.

Computer processing system 150 as illustrated in Figure 1 is a general purpose computer processing system. It will be appreciated that the particular type of computer processing system will determine the appropriate hardware and architecture, and alternative computer processing systems suitable for implementing aspects of the invention may have additional, alternative, or fewer components than those depicted. The computer processing system 150 includes at least one processing unit 152. The processing unit 152 may include a single computer processing device (e.g. a microprocessor or other computational device), or may include a plurality of computer processing devices. In some instances all processing will be performed by processing unit 102, however in other instances processing may also, or alternatively, be performed by remote processing devices accessible and useable (either in a shared or dedicated manner) by the system 100.

Through a communications bus 154 the processing unit 152 is in data communication with a system memory 156 (e.g. a BIOS), volatile memory 158 (e.g. random access memory including one or more DRAM modules), and non-volatile memory 160 (e.g. one or more hard disk drives, solid state drives, and/or ROM devices such EPROMs). Instructions and data for controlling operation of the processing unit 152 are stored on the system, volatile, and/or non-volatile memory 156, 158, and 160.

Computer processing system 150 also includes one or more input/output interfaces (indicated generally by 162) which interface with a plurality of input/output devices. As will be appreciated, a wide variety of input/output devices may be used, including intelligent input/output devices having their own memory and/or processing units. By way of non-limiting example, the system 150 may include or connect to: one or more user input devices 164 (e.g. keyboard, mouse, a touch-pad, trackpad, microphone, etc.); one or more user output devices 166 (e.g. CRT display, LCD display, LED display, plasma display, touch screen display, speaker, etc.); one or more ports 168 for interfacing with external devices such as portable device 100, drives, and/or memory (e.g. via USB ports, Firewire ports, eSata ports, serial ports, parallel ports, SD card port, Compact Flash port, etc.); and one or more communications interfaces 170 for communicating data to and/or receiving data from another device or networked location (e.g. a Network Interface Card, a Bluetooth module, an infrared module, a WiFi module).

The computer processing system 150 will run one or more applications to allow a user to operate the system 150. Such applications will typically include at least an operating system (such as Microsoft Windows®, Apple OSX, Apple IOS, Android, Unix, or Linux). Communication between the portable device 100 and a computer processing system such as 150 may be direct communication (e.g. via a USB or other connection), or may be indirect via a communications network 122 (e.g. the Internet, a personal area network, local area network, wide area network or other network).

Computer system 150 may be any suitable computer processing system such as, by way of non-limiting example, a desktop computer, a laptop computer, a netbook computer, tablet computer, a smart phone, a PDA.

As described below, embodiments of the present invention may also make use of third-party computer processing systems such as 180 to store data on, retrieve data from, and/or process data. Such third-party systems 180 will typically be accessed over a network such as the Internet and themselves be computer processing systems having similar functional components as computer processing system 150.

### Processing data to automatically identify points/times of interest

Activity data collected by the portable device 100 is processed by a computer processing system in order to provide the user with various information and visualisations concerning their activity. Processing of the data will be described in the context of computer-implemented methods. Such methods are performed by software which include computer readable instructions which are executed by a computer processing unit (such as 106 or 152) to implement the relevant functionality. The instructions may be conveyed to a computer processing system by means of a data signal in a transmission channel. Examples of such transmission channels include wired or wireless network connections enabled by a communications interface (e.g. 170) and various communications protocols.

It will be appreciated that in some cases part or all of a given computer-implemented method may be performed by the portable device 100 itself (provided, of course, the portable device 100 includes the appropriate hardware and software). In other cases part or all of a computer implemented method may be performed by a separate computer processing system such as system 150 which has received activity data from the portable device 100.

In the sport of surfing, information in respect of a surf session that is of particular interest includes the periods of time the surfer is actually surfing (i.e. riding a wave), as distinct from time periods when the surfer is not surfing (e.g. paddling out or waiting to catch a wave).

Existing systems for running and cycling allow users to manually set markers to indicate points (times) of interest over the course of the activity. For example, if a cyclist or runner stops at a set of lights they may manually activate a stop control on their portable device to indicate that the device should stop recording data. When the light turns green, the runner/cyclist can activate a start control to resume recording data. Alternatively, a cyclist/runner may activate a "lap" control or similar to mark one or more particular times over the course of the activity.

In the surfing context, however, manually activating controls on a portable device to record markers indicating start and end times for surfing a wave is highly impractical. When trying to catch a wave the surfer is first using their hands and arms to paddle, then using their hands/arms to transition from paddling to standing, then using their hand/arms to balance while riding the wave. Similarly, when finishing a wave the surfer is using their hands/arms to paddle out of the wave (or, in the case of wiping out, to reach the surface). These do not, practically speaking, allow for the manual activation of controls on the portable device 100 to record when a wave is caught and when the wave is left.

In one arrangement, the present invention provides a computer-implemented method for processing positional (e.g. GPS) data recorded by a portable device 100 over the course of a surfing session to automatically determine when a surfer is surfing on a wave and when the surfer is not surfing on a wave. To achieve this, and as described in detail below, the activity data is processed to estimate wave start points (indicative of when a surfer has caught a wave) and wave end points (indicative of when a surfer has left a wave). This allows time and activity data relating to actual surfing time (and to individual waves caught) to be easily distinguished from time and activity data not relating to actual surfing time.

### Recording track points

The GPS receiver 102 of the portable device 100 receives GPS signals emitted from orbiting GPS satellites. These GPS signals are used to calculate information such as the position and speed of the portable device 100 (and, accordingly, the person carrying or wearing the portable device 100) over the course of their activity.

For example, the GPS receiver 102 may be configured to sample positional information received from the GPS satellites at "ping" rate (e.g. four hertz). Based on this positional information speed can be calculated, for example using a 64 point (16 seconds for a ping rate of four hertz) weighted averaging algorithm. While all received/calculated data can be used for downstream processing, in one embodiment portable device 100 is configured to calculate and record average position and speed data at a lower rate than the ping rate for downstream processing. For example, device 100 may store averaged values for each second (i.e. by averaging the four values actually for that second). By storing averages of the speed and position data actually received, the amount of memory required is reduced as is the amount of data that needs to be transferred from the portable device 100 to another computer processing system 150.

The GPS activity data recorded to memory and used for downstream processing will be referred to as track points. Table 1 below illustrates how speed track points are calculated from GPS data received over a 3 second period (in this case the ping rate is 4 hertz and track points are calculated/stored at each 1 second interval):

**Table 1**

| **Time (seconds)** | **Speed (km/h) (e.g. 64 point weighted average)** | **Trackpoint Average speed (km/hr.)** |
|---|---|---|
| ¼ | 10 | 8.5 |
| ½ | 9 | |
| ¾ | 8 | |
| 1 | 7 | |
| 1¼ | 11 | 8.75 |
| 1½ | 9 | |
| 1¾ | 8 | |
| 2 | 7 | |
| 2¼ | 8 | 6 |
| 2½ | 6 | |
| 2¾ | 5 | |
| 3 | 5 | |

Other information that may be received and/or calculated and stored by the portable device 100 at each track point may include (provided appropriate monitoring/sensor equipment is operating) satellite information, heart rate information, longitude information, latitude information, altitude information, heading information, and distance travelled. Table 2 below illustrates an example of activity data that may be stored at each track point:

**Table 2**

| Id | Time | Satellite | Heart rate | Speed (km/h) | Longitude | Latitude | Altitude (m) | Heading | Distance (km) |
|---|---|---|---|---|---|---|---|---|---|
| 1 | 24/02/2014 13:39 | 7 | 103 | 0 | 144.284525 | -38.36817 | 10 | 12 | 0 |
| 2 | 24/02/2014 13:39 | 7 | 103 | 0.5 | 144.284525 | -38.36817 | 10 | 12 | 0 |
| 3 | 24/02/2014 13:39 | 7 | 103 | 0.7 | 144.2845233 | -38.36817 | 10 | 12 | 0 |
| 4 | 24/02/2014 13:39 | 7 | 103 | 0.8 | 144.2845233 | -38.3681716 | 10 | 12 | 0 |
| 5 | 24/02/2014 13:39 | 7 | 103 | 0.8 | 144.2845233 | -38.3681716 | 10 | 12 | 0 |
| 6 | 24/02/2014 13:39 | 7 | 103 | 0.8 | 144.2845233 | -38.3681733 | 10 | 12 | 0 |
| 7 | 24/02/2014 13:39 | 7 | 103 | 0.9 | 144.2845233 | -38.3681733 | 10 | 12 | 0 |
| 8 | 24/02/2014 13:39 | 7 | 103 | 0.9 | 144.2845233 | -38.3681733 | 10 | 12 | 0 |
| 9 | 24/02/2014 13:39 | 7 | 103 | 0.8 | 144.2845233 | -38.368175 | 10 | 12 | 0 |
| 10 | 24/02/2014 13:39 | 7 | 103 | 0.8 | 144.2845233 | -38.368175 | 10 | 12 | 0 |

In other embodiments, potentially in the context of another sporting activity, the portable device 100 may be configured to calculate speed based on an alternative satellite ping rate and/or storage interval, depending on factors such as the expected speed ranges of the user.

### Activity markers

In order to estimate wave start and wave end points, activity track points are processed according to wave recognition criteria. Track points/data falling between a consecutive pair of wave start and end points relate to time during the activity when the user was actually surfing a wave. Conversely, track points/data falling between a consecutive pair of wave end and wave start points relate to time during the activity when the user was not actually surfing a wave, e.g. paddling or floating.

As noted, processing of the activity data to determine wave start and wave end points may in some cases be performed on the portable device 100 itself (potentially in real time). In other cases the processing may alternatively (or additionally) be performed on an alternative computer processing system (such as 150) based on activity data obtained from the portable device 100.

### Speed-based determination

In one embodiment, the wave recognition criteria used to determine wave start points and wave end points is based on the user's speed. In this embodiment, a wave start point is logged when speed exceeds a predetermined on-wave speed threshold for longer than a predetermined on-wave time window. Although catching the wave is not identified until the end of the predetermined on-wave time window (i.e. until the speed has exceeded the predetermined on-wave speed for the duration of the on-wave time window), the wave start point is recorded as being the track point at the beginning of the relevant on-wave time window.

Once a wave start point has been determined, the processor may be configured to not to analyse the data to detect any further wave start points while the speed since the wave start point has been maintained above a predetermined riding speed threshold. Once a wave start point has been determined, the processor may also be configured to not to analyse the data to detect any further wave start points until after a wave end point is recorded.

In this embodiment a wave end point is logged when speed falls below a predetermined off-wave speed threshold for longer than a predetermined off-wave time window. Once again, although the end of the wave is not identified until the end of the predetermined off-wave time window (i.e. until the speed has remained below the predetermined off-wave speed for the duration of the off-wave time window), the wave end point is recorded as being the track point at the beginning of the relevant off-wave time window.

Once a wave end point has been determined, the processor may be configured to not to analyse the data to detect any further wave end points until after a wave start point is recorded.

After detecting a wave end point, the system may also be configured to not record a new wave start point (or not analyse data for a new wave start point), until a predetermined lock-out period since the last identified wave start point has elapsed. The lock-out period is set based on the amount of time it is considered impractical for a surfer to catch a new wave after having identified that the surfer has started a preceding wave.

Over the course of the activity, a number of consecutive (based, for example, on the timing of the wave start and wave end track points) wave start and wave end points will typically be identified. Together, a consecutive pair of wave start and wave end points define a wave, and indicate that the data recorded between those two track points relates to when a surfer is on the wave.

In one specific (though non-limiting) implementation, the following thresholds/time windows may be used. The pre-determined on-wave speed threshold may be 2.7 m/s and the predetermined on-wave time window may be 6 seconds. In other words, if the track points show that the surfer was travelling at greater than 2.7 m/s consistently for over 6 seconds the processor will determine that the surfer caught a wave at the time of the track point at the start of the 6-second window. The pre-determined off-wave speed threshold may also be 2.7 m/s and the predetermined off-wave time window may be 3 seconds. In other words, if the track points show that (following a wave start point being detected) the surfer has been travelling at less than 2.7 m/s consistently for over 3 seconds the processor will determine that the surfer left a wave at the time of the track point at the start of the 3-second window. The predetermined riding speed threshold may be set at 10km/h. The lock-out period may be 30 seconds.

Alternative on-wave speed thresholds, on-wave time windows, off-wave speed thresholds, off-wave time windows, riding speed thresholds and lock-out periods are, of course, possible. Further, the on-wave and off-wave speed thresholds may be the same or different speeds, and/or the on-wave time and off-wave time windows may be the same or different time periods.

Additionally, the predetermined on-wave and/or off-wave speeds may be set depending on surf conditions and or location. For example, a higher predetermined on-wave and/or off-wave speed may be used for larger waves.

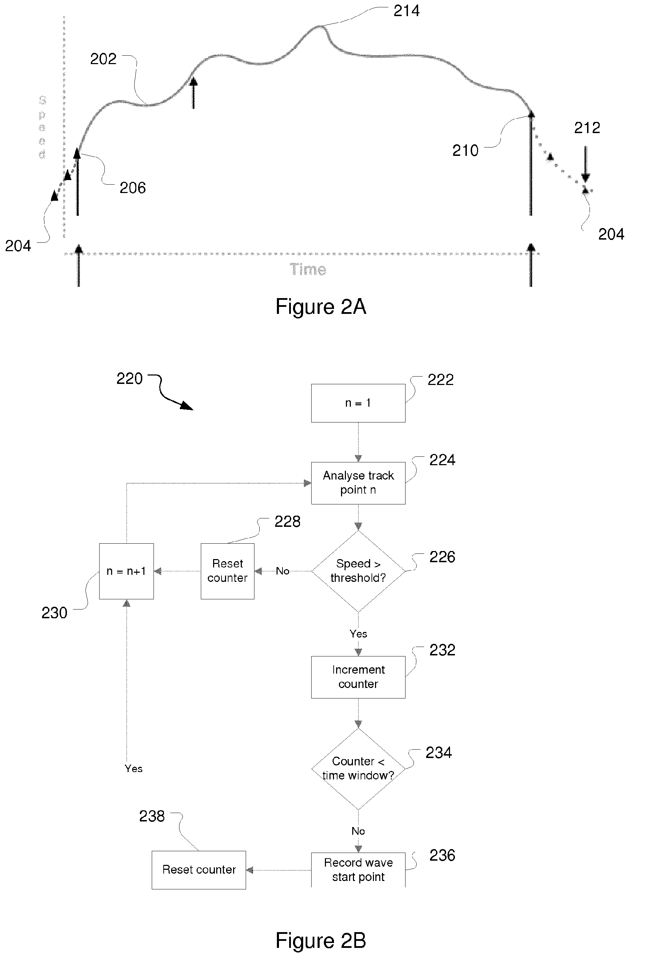

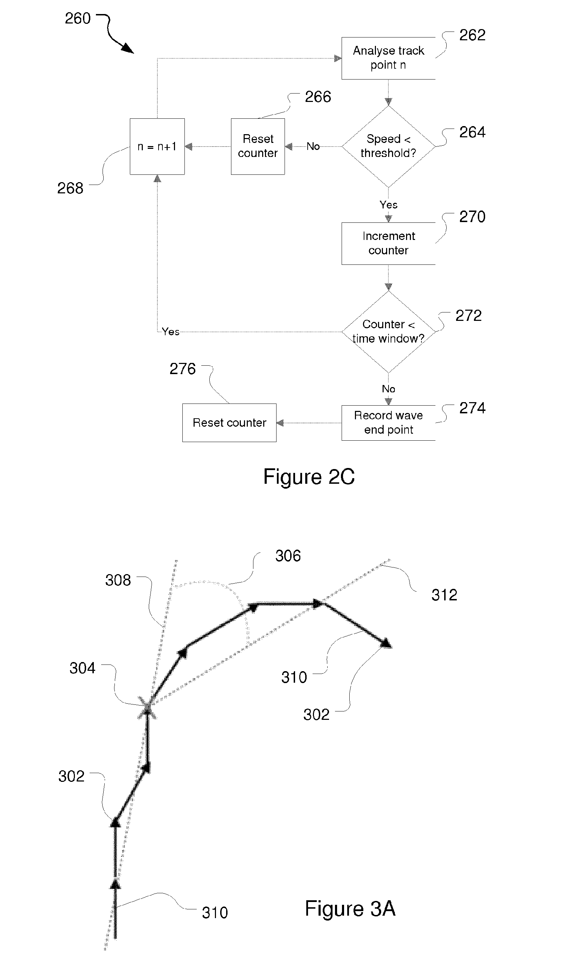

Figure 2A illustrates schematically an example of using the speed based wave recognition criteria described above to determine a wave start point and a wave end point. Line 202 is fit-line extrapolated between speed track points 204 of the activity data. Solid portions of line 202 indicate time detected to be on the wave (i.e. between a consecutive pair of wave start and end points), while broken outline portions of line 202 indicate time not spent on the wave. At point 206, the speed of the surfer exceeds the on-wave speed threshold. At 208, the speed of the surfer is detected to have remained above the on-wave speed threshold for duration of the on-wave time window. At this point the surfer is considered to have caught a wave, and to have been surfing on the wave since the start of the on-wave time window - i.e. a wave start point is set as the time of point 206. At point 210 the speed of the surfer falls below the off-wave speed threshold, and at point 212 the speed of the surfer is detected to have remained below the off-wave speed threshold for duration of the off-wave time window. At this point the surfer is considered to have left the wave (i.e. no longer be surfing), and to have left the wave at the start of the off-wave time window - i.e. a wave end point is set as the time of point 210. (In the example of Figure 2 the on-wave speed threshold and off-wave speed thresholds are different.) Track points/data between the wave start point at 206 and the wave end point 210 is therefore treated as data relating to actual surfing. At point 214 the surfer is considered to attain a top speed during a particular surf.

Figure 2B provides a flowchart of steps involved in one particular method 220 for analysing activity data to determine wave start points according to the speed based method above. Alternative algorithms are, of course, possible.

In method 220, a current track point variable n and an on-wave counter variable (initialised to 0) are maintained. For convenience, track points are assumed to be numbered in chronological order from track point 1 to track point x.

At step 222, the index of the first track point in the activity data to be analysed is set. In this instance track point 1 (n=1) is selected.

At step 224 the current track point (track point n) is analysed.

At step 226 the speed recorded for the current track point is compared with the on-wave speed threshold.

At step 228, if the speed recorded for the current track point is less than or equal to the on-wave speed threshold, the on-wave counter variable is set to 0. The track point index is then incremented (n=n+1) at step 230 and the process returns to step 224.

At step 232, if the speed recorded for the current track point is greater than the on-wave speed threshold, the on-wave counter variable is incremented by 1.

At step 234, the on-wave counter variable is compared against a value representing the length of the predetermined on-wave time window.

The predetermined on-wave time window can be reduced to a simple integer value according to the track point sampling rate and the duration of the window. For example, if track points are recorded every x seconds (or xth of a second), and the on-wave time window is y seconds, an on-wave counter value of ((1/*x*)**y*) + 1 will indicate that the surfer's speed has exceeded the on-wave speed threshold for the predetermined on-wave time window. By way of more specific examples:
- if track points are sampled every 1 second and the on-wave time window is 10 seconds, an on-wave counter value of ((1/1)*10)+1 = 11 will indicate a wave has been caught;
- if track points are sampled every 1/2 a second and the on-wave time window is 10 seconds, an on-wave counter value of ((1/(1/2)*10)+1 = 21 will indicate a wave has been caught.

If the on-wave counter is less than the predetermined on-wave time window, the next track point in the activity data (track point n+1) is selected (step 230) and the process returns to step 224.

At step 236, if the on-wave counter has reached the predetermined on-wave time window, the relevant track point is flagged as a wave start point. The relevant track point is the track point at the start of the predetermined on-wave time window. In the present case where track points are indexed numerically, the track point to be flagged as the wave start point can be calculated as track point with the index of: *n -* the value of the counter + 1 (with n being the index of the current track point).

At step 238 the on-wave counter is reset to 0.

The determination of a wave end point can be made in a similar manner to the determination of a wave start point. Figure 2C provides a flowchart of steps involved in one particular method 260 for analysing activity data to determine wave end points according to the speed based method above. Alternative algorithms are, of course, possible.

In method 260, a current track point variable n and an off-wave counter variable (initialised to 0) are maintained. For convenience, track points are again assumed to be numbered in chronological order.

At step 262 the current track point (track point n) is analysed. In one case method 260 may continue on from method 220, in which case the current track point will be the next track point after a wave start point is recorded at step 236. (I.e., method step 262 may follow on from method step 238 of method 220 with an intervening track point index increment (n=n+1) being made.

At step 264 the speed recorded for the current track point is compared with the off-wave speed threshold.

At step 266, if the speed recorded for the current track point is greater than or equal to the off-wave speed threshold, the off-wave counter variable is set to 0. The track point index is then incremented (n=n+1) at step 268 and the process returns to step 262.

At step 270, if the speed recorded for the current track point is less than the off-wave speed threshold, the off-wave counter variable is incremented by 1.

At step 272, the off-wave counter variable is compared against a value representing the length of the predetermined off-wave time window. The predetermined off-wave time window can be reduced to a simple integer value in a similar manner to that described above with respect to the predetermined on-wave time window.

If the off-wave counter is less than the predetermined off-wave time window, the next track point in the activity data (track point n+1) is selected (step 268) and the process returns to step 262.

At step 274, if the off-wave counter has reached the predetermined off-wave time window, the relevant track point is flagged as a wave end point. The relevant track point is the track point at the start of the predetermined off-wave time window.

At step 276 the off-wave counter is reset to 0.

Having determined a wave end point, further track points may then be analysed for the next wave start point. For example, after step 276 the track point index may be incremented (n=n+1) and the method recommence at step 224 of method 220.

### Angular deviation based determination

In an alternative embodiment, the wave recognition criteria used to determine the wave start point and the wave end point may include criteria based on the surfer's path. Figure 3A provides an illustration of this methodology. In this embodiment the track points 302 are processed to determine whether the surfer's path before and after a given track point deviates by more than a predetermined on-wave angle (to define a wave start point) or a predetermined off-wave angle (to define a wave end point).

The angular deviation of the surfer's path at a particular track point 304 may be calculated as the angle 306 at which a first straight line of best fit 308 through first group of several consecutive path segments 310 (each path segment 310 a straight line joining consecutive track points 302 leading up to the track point 304 in question) joins a second straight line of best fit 312 through a second group of several consecutive path segments 310 (joining consecutive track points 302 following the track point 304 in question). A sufficiently large angle of deviation 306 between the first and second straight lines 308 and 312 is interpreted that the surfer has either turned to align with a wave (e.g. to catch the wave - in which case a wave start point is recorded) or has turned to cross a wave (e.g. to leave the wave - in which case a wave start point is recorded).

In one specific embodiment the pre-determined on-wave and off-wave angles are 40 degrees, and the first and second straight lines 308 and 312 are calculated from three track points (i.e. three path segments) prior to the deviation point 304 and three track points after the deviation point 304.

The angular deviation based wave recognition criteria may also include criteria based on the distance travelled by the surfer near the deviation point 304 - e.g. in the lead-up to the deviation point 304 and/or the follow-on to the deviation point 304.

For example, the criteria may specify that a wave start or wave end point is not recognised or recorded unless the surfer has travelled by more than a threshold lead-up distance to the deviation point 304 within a lead-up time period (i.e. a set number of track points). In this example, the number of track points 302 corresponding to the lead-up time period are processed to determine whether the surfer has travelled by more than the threshold lead-up distance within the lead-up time period.

Additionally or alternatively, the criteria may specify that the wave start point or wave end point is not recognised or recorded unless the surfer has travelled by more than a threshold follow-on distance to the deviation point 304 within a follow-on time period (i.e. a set number of track points). In this example, the number of track points 302 corresponding to the follow-on time period are processed to determine whether the surfer has travelled by more than the threshold follow-on distance within the follow-on time period.

The lead-up threshold distance and/or lead-up time period may be the same as or different from the threshold follow-on distance and/or the follow-on time period. In one embodiment, the lead-up and follow-on distance thresholds are both 2.4 meters and the lead-up and follow-on time periods are both three seconds (i.e. three track points when track points are created for each second).

In Figure 3A, the follow-on path is represented by the three arrows (track points) forward-tracing from the deviation track point 304 and the lead-up path is represented by the three arrows (track points) back-tracing from the deviation point 304.

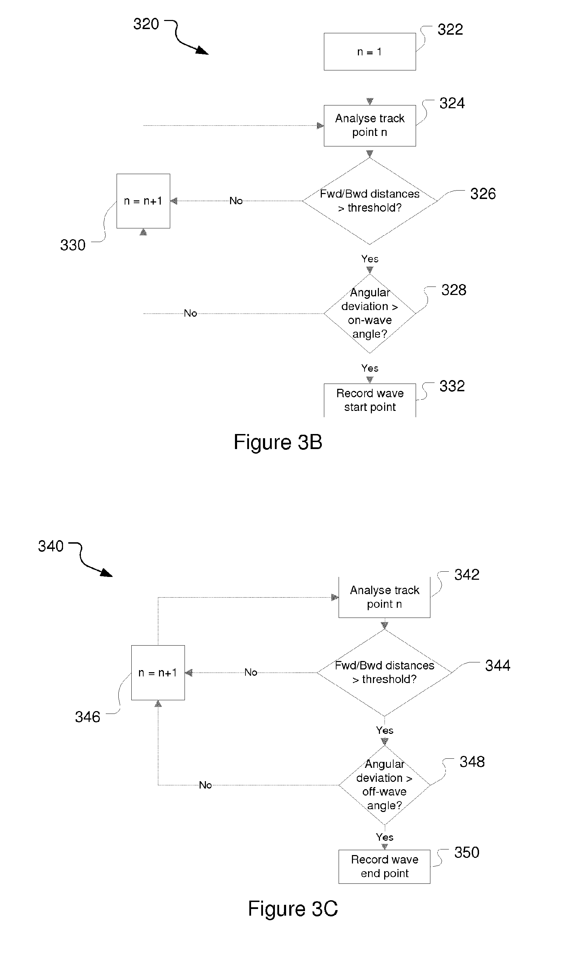

Figure 3B provides a flowchart of steps involved in one particular method 320 for analysing activity data to determine wave start points according to the angular deviation based method above. Alternative algorithms are, of course, possible.

In method 320, a current track point variable n is maintained. For convenience, track points are assumed to be numbered in chronological order from track point 1 to track point x.

At step 322, the index of the first track point in the activity data to be analysed is set. In this instance track point 1 (n=1) is selected.

At step 324 the current track point (track point n) is analysed.

At step 326 the follow-on distance and lead-up distances are calculated from the recorded data and compared with the threshold follow on and lead-up distances. The follow-on distance is calculated according to the distance travelled between the current track point and the number of following track points corresponding to the follow-on time period (e.g. 3 track points in the case that the time period is 3 seconds and track points are recorded every second). Similarly, the lead-up distance is calculated according to the distance travelled between the current track point and the number of preceding track points corresponding to the lead-up time period.

If either or both of the follow-on and lead-up distances to the current track point are determined to be less than or equal to the relevant threshold distances, the next track point is set as the current track point at step 330 and the process returns to step 324.

If both the follow-on and lead-up distances for the current track point are determined to be greater than the relevant threshold distances, the process continues to step 328.

At step 328, the angular deviation between the follow-on the lead-up paths is compared with the on-wave angle. If the angular deviation is less than or equal to the on-wave angle, the next track point is set as the current track point at step 330 and the process returns to step 324. If the angular deviation is greater than the on-wave angle, the process continues to step 332.

At step 332, the current track point is recorded as a wave start point.

The determination of a wave end point can be made in a similar manner to the determination of a wave start point. Figure 3C provides a flowchart of steps involved in one particular method 340 for analysing activity data to determine wave end points according to the angular deviation based method above. Alternative algorithms are, of course, possible.

In method 340, a current track point variable n is maintained. For convenience, track points are assumed to be numbered in chronological order from track point 1 to track point x.

At step 342 the current track point (track point n) is analysed. In one case method 340 may continue on from method 320, in which case the current track point will be the next track point after a wave start point is recorded at step 332. I.e., method step 342 may follow on from method step 332 of method 320 with an intervening track point index increment (n=n+1) being made.

At step 344 the follow-on distance and lead-up distances are calculated from the recorded data and compared with the threshold follow on and lead-up distances. This can be done in the same or a similar manner to that described with respect to method 320.

If either or both of the follow-on and lead-up distances to the current track point are determined to be less than or equal to the relevant threshold distances, the next track point is set as the current track point at step 346 and the process returns to step 342.

At step 348, if both the follow-on and lead-up distances for the current track point are determined to be greater than the relevant threshold distances, the angular deviation between the follow-on the lead-up paths is compared with the on-wave angle. If the angular deviation is less than or equal to the on-wave angle, the next track point is set as the current track point at step 346 and the process returns to step 342.

If the angular deviation is greater than the on-wave angle, the process continues to step 350 and the current track point is recorded as a wave end point.

### Composite determination

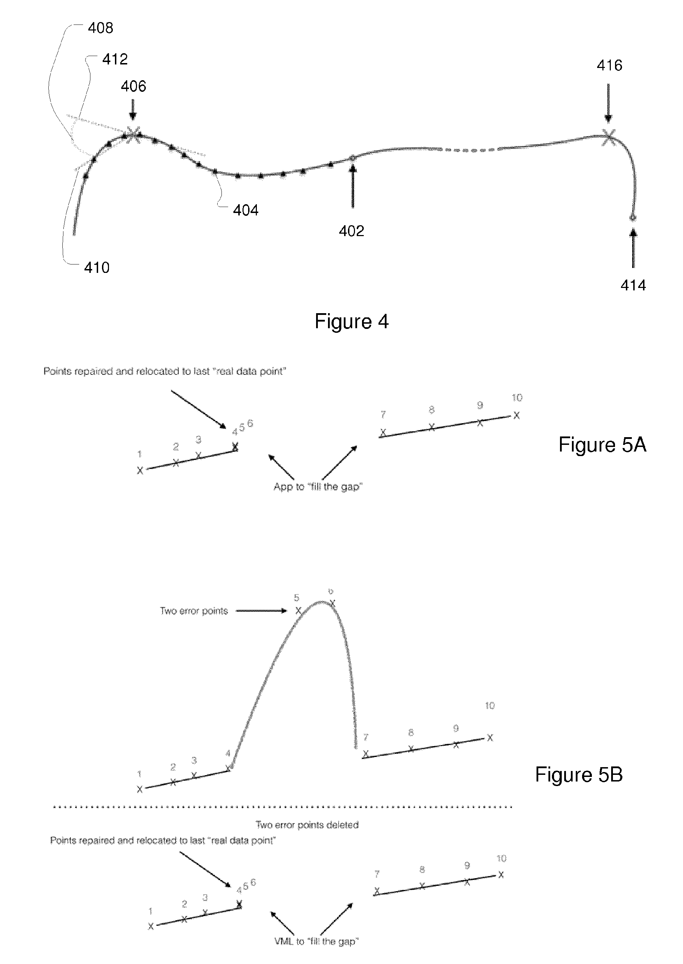

In a further embodiment, processing the activity data to determine wave start and end points may involve a consideration of both the speed and angular deviation of the surfer. This embodiment is illustrated in Figure 4.

In this case, the activity data is initially analysed using the speed determination methodology described above. If a preliminary wave start point 402 is identified using the speed determination methodology, a set number of preceding track points 404 (in this particular example 12 preceding track points) are analysed using the angular deviation methodology described above. If analysis of the preceding track points using the angular deviation methodology indicates an earlier wave start point 406 occurred (due to the angular deviation 408 between lines 410 and 412 exceeding the on-wave angular threshold), the preliminary wave start point 402 is overridden in favour of the earlier wave start point 406 determined using the angular determination methodology.

Similarly if a preliminary wave end point 414 is identified using the speed determination methodology described above a set number of preceding track points are analysed using the angular deviation methodology described above. If analysis of the preceding track points using the angular deviation methodology indicates an earlier wave end point 416 occurred, the preliminary wave end point 414 is overridden in favour of the earlier wave end point 416 determined using the angular determination methodology.

### Additional data and criteria

In further embodiments, the activity data may include acceleration data obtained from one or more accelerometers. Such accelerometers may be included in device 100. In this case acceleration data can also be used in the wave recognition criteria to assist the determination of when a user is surfing a wave.

For example, acceleration data indicating the user has repeatedly turned left and right may indicate the user is riding a wave rather than, for example, paddling. Similarly, if the accelerometers are included in the portable device, and the device is a watch to be worn on the surfer's wrist, the action of paddling may be easily distinguishable from the action of surfing.

Further rules based on the available data may also be applied to more accurately estimate whether a surfer is on a wave or not. For example, acceleration, speed, and/or positional data may be used to determine that the surfer is actually on the beach

(walking or running) rather than in the water. In this case the data will either not be processed in order to determine wave start and end points (until the surfer is determined to have entered the water), or any wave start/end points identified will be disregarded.

There may be also circumstances where, despite the relevant wave recognition criteria being satisfied, a wave is not registered. By way of example, some of these circumstances include:
- The user's recorded speed is over a speed considered dangerous.
- The user's recorded speed is considered too fast to be obtainable in the activity.
- A wave is determined within a set time of a previous wave being registered.
- The stored data at a track point is not associated with a 3D lock (e.g. more than 4 satellites) on GPS signal.
- A track point has been recorded with a horizontal dilution of pollution (HDOP) above a set value (and is therefore considered an "error point").

### GPS dropout or error points

Referring to Figure 5A, if there is a GPS signal dropout, the position of a track point during the dropout is held at last known track point. Once the GPS signal is found again, data associated with the lost track points may be calculated by assuming an average velocity (i.e. speed and direction) of the user during the dropout, and interpolating between the known track points, before and after the dropout. The lost track points may be associated with paddling by the user instead of surfing.

Similarly, referring to Figure 5B, if any erroneous track points are identified (e.g. due to exceeding the HDOP threshold), these may be repaired/corrected in a similar manner to the way in which lost track points are interpolated.

### Processing, display, and sharing of information

By identifying wave start and end points, data recorded in a session can be identified as data relating to surfing (i.e. any data falling between consecutive pair of wave start and wave end points) and data not related to surfing (i.e. data falling between a consecutive pair of wave end and wave start points). This, in turn, allows the activity data to be processed to provide additional metrics/information of use or interest to a user in respect of a surfing session (or in respect of a number of surfing sessions).

One aspect of the invention provides a user application or software for recording, processing and displaying information relating to a user's activity sessions. As discussed below, the application accesses and stores the raw activity data as recorded by the portable device 100 as well as additional information including data calculated based on the raw data, data obtained from third party providers, and data manually entered by the user. By storing this information the application maintains a detailed surf log of the user's surf sessions.

The user application may be an application stored in memory of a device owned by the user (e.g. a computing system such as 150) and run locally, or may be a web application hosted by a third party and accessible to the user over the Internet. Wherever the application is run it is provided with access to the raw activity data recorded by the portable device (e.g. by transfer of the activity data from the portable device 100 to a relevant computer system). Using this activity data the application can include instructions for performing the processing described above (e.g. in order to determine wave start and end points), as well as additional processing to obtain additional information and allow a user to view, manipulate and/or share information relating to their activities.

In some cases the user application (or a cut-down version thereof offering a subset of the broader functionality) may be installed and run on the portable device 100 itself. Typically, however, the application will be run on an alternative computing system such as system 150. This is due to the increased capabilities such a system will typically have when compared to the portable device 100 (in terms of processing power, screen display area, network connectivity, memory and the like). Even where the application can be run on the portable device 100 itself, users will typically want to periodically transfer their data to another computer system (either owned by the user or in the cloud) to keep a record of their data.

The application provides a user interface allowing a user to determine and view various information regarding one or more of their surfing sessions. Such information may include, for example:
- The number of waves caught (i.e. the number of start wave points recorded) for the day, the last 28 days, year to date or since activity data have been recorded.
- The time at which each wave was caught (i.e. the start wave point for each wave).
- The top speed recorded (i.e. the top speed recorded over the surfing session).
- The longest wave caught (i.e. the longest distance travelled between consecutive wave start and wave end points).
- The total distance travelled while surfing on waves (i.e. the cumulative distance travelled between all consecutive pairs of wave start and wave end points).
- The total distance paddled (i.e. the cumulative distance travelled between all consecutive pairs of wave end and wave start points).
- The amount and/or percentage of time spent actually on waves (i.e. the cumulative time elapsed between all consecutive pairs of wave start and wave end points).
- The amount and/or percentage of time during the session spent not on waves (i.e. the cumulative time elapsed between all consecutive pairs of wave end and wave start points).
- For each wave, the top speed recorded whilst on the wave (i.e. the top speed recorded between the consecutive wave start and wave end points defining the wave).
- For each wave, the distance travelled on the wave (i.e. the distance travelled between the consecutive wave start and wave end points defining the wave).
- For each wave, the location of the wave (i.e. the GPS position at the start point of the wave).
- For each wave, the path travelled whilst on the wave (i.e. the GPS positions recorded between the consecutive wave start and end points defining the wave).

The user interface provided by the application allows the user to view/calculate/process such information either in respect of a single surf session, a selection of available surf sessions, or all available surf sessions. For example, a user may only want to see information related to their most recent session, or may want to see cumulative information (e.g. waves caught, distances travelled etc.) for all sessions that took place in 2014 (or all sessions the user has recorded).

The application also operates to access and retrieve data from one or more third party providers (e.g. data provided on systems 180). The application may automatically obtain such data (e.g. every time activity data is captured or uploaded to the computer processing system and the relevant application is launched), or may provide a control operable by a user to cause the application to retrieve such data on demand.

By way of example, map and/or satellite image data in respect of the location of a surfing session may be accessed/retrieved from an appropriate map data provider (e.g. Google Maps). The application can then display the map or satellite image data to the user with the actual paths of waves caught and/or paddling overlaid thereon.

By way of further example, the application may be configured to access (automatically or on demand) or calculate surf and/or weather condition data relevant to the location and time of the surf session from an appropriate surf information provider. This additional information may also be recorded in the user's surf log. One such provider (as at 16 May 2014) is Magic Seaweed which makes surf-related data available via its website (magicseaweed.com). Using the time and location information of a given surf session (as obtained from the portable device 100), the application may make appropriate API calls to the server of such a data provider in order to retrieve additional data relating to a given surfing session. Such data may include, for example:
- Average wave height at the surf location and time indicated by the activity data.
- Average period between waves at the surf location and time indicated by the activity data.
- Average wind air speed and direction at the surf location and time indicated by the activity data.
- Wind gust speed and direction at the surf location and time indicated by the activity data.
- Tide direction and height at the surf location and time indicated by the activity data.
- Tide direction and height at the surf location and time calculated by extrapolating historical tide data obtained, for example, as described above.

As data from such providers is not typically obtained from first-hand observations, the user interface provided by the application allows the obtained information to be manually overridden by the user. For example, if user is viewing information regarding a session where they know the wave height was 3 meters, but where the information obtained from the surf information provider indicates the wave height was 2 meters, the user can correct the value to match their experience/observations.

Further, the application may allow the user to manually enter data relating to their surfing sessions into their surf log. For example, a rating field may be provided by which a user can rate a given surf session (e.g. by a number rating system, a star rating system or similar). One or more comment fields may also be provided in which the user can enter comments related to a particular surf session. The application may also allow the user to store (or link to) one or more photographs/images relating to a surf session. A field may also be provided to allow the surfer to record the particular surf board used for the surfing session.

The application may also allow a user to enter and store user profile information, for example the surfer's preferred stance, surfboard(s) owned/used by the surfer, height, age, weight, gender and/or one or more photographs.

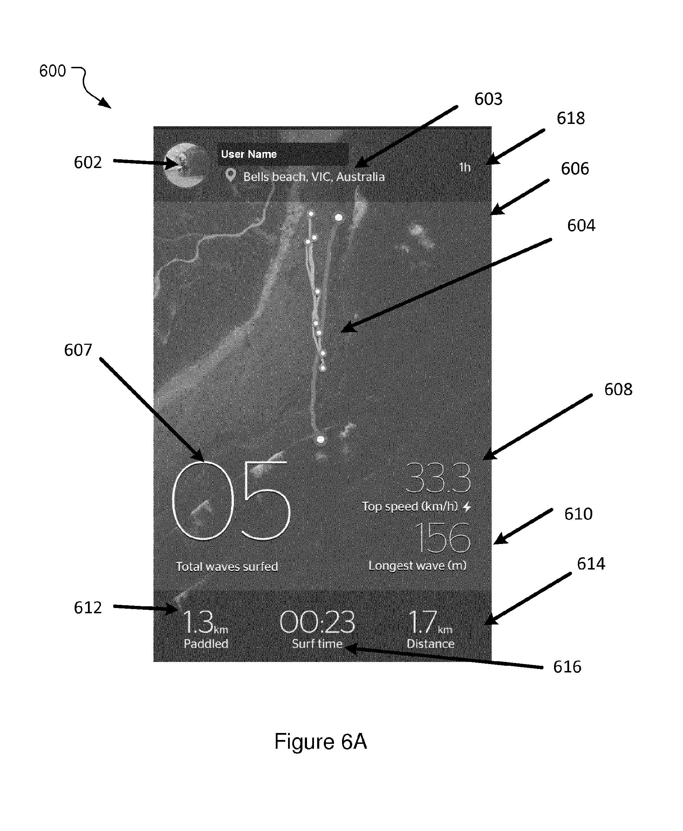

Figures 6A to 6D provide example user interface screens generated by the application to allow users to view and enter data relevant to surf sessions and their user profile. In this instance the user interface screen 600 is appropriate for a smart phone with a touch screen display.

Figure 6A depicts a user interface screen 600 which displays the following information in respect of a surfing session:
- User account information 602 (indicating in this case the name of the user and a profile image related to the user account being viewed);
- Location information 603 indicating the location of the surf session (in this case Bells Beach, Victoria Australia).
- Wave lines 604 showing the positions of five waves that were identified as being caught during a surfing session. Such wave lines are generated by linking and smoothing location track points recorded between consecutive wave start and end points.

In this instance the wave lines 604 are displayed on a satellite image 606 of the area in which the surfing session took place (obtained, for example, from a map data provider). The application may also allow for the wave lines 604 to be displayed on a stylised area map, or on no background (in which case the wave lines 604 are simply displayed so as to show their locations relative to one another).
- A total number of waves surfed 607 (five in in this instance).
- A top speed 608 (33.3 km/h in this instance).
- The longest wave caught 610 (156m in this instance).
- The total distance paddled 612 (1.3km in this instance).
- The total distance 614 surfed (1.7km in this instance).
- The duration 616 of the time spent surfing in the surf session (23 minutes in this instance).
- The duration 618 of the total surf session (1 hour in this instance).

Additional information could also be shown, for example paddle lines may also be displayed to indicate track points representing paddling by the user.

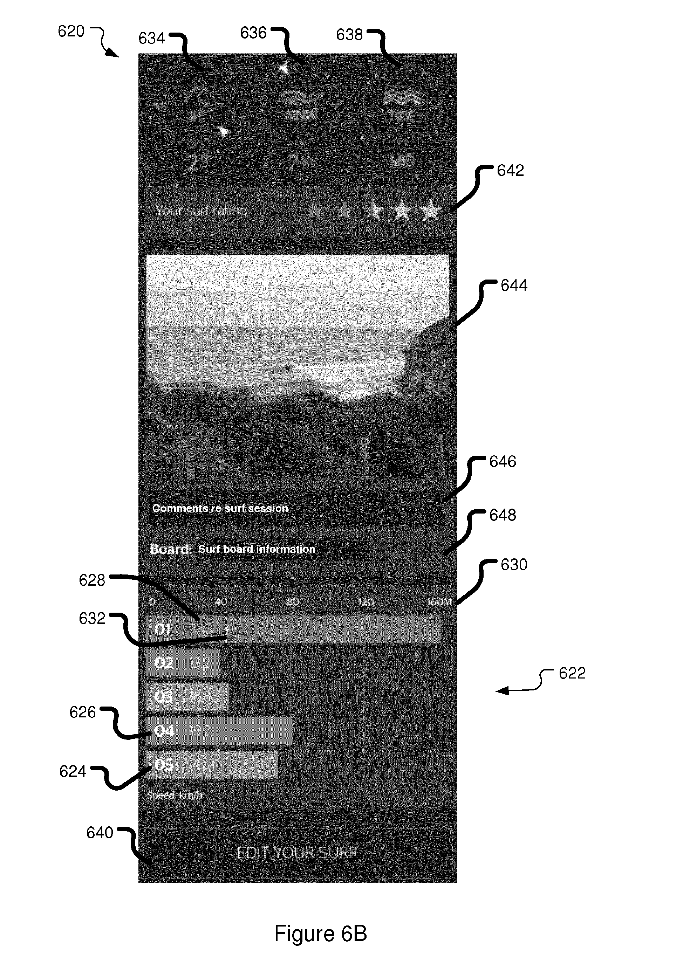

Figure 6B provides an example of a second user interface screen 620 generated by the application and providing information regarding the a surfing session. Rather than displaying wave lines of the waves caught in the surfing session (as per interface screen 600), interface screen 620 displays the waves caught 622 in a bar-chart type fashion in which each wave caught is displayed as a bar 624 having a length indicating the distance travelled on the wave.

Each bar 624 (indicating a wave caught) includes the number of the wave in the surfing session 626 (e.g. the 4th wave caught in the session) and the top speed reached whilst on the wave 628 (for wave four 19.2 km/h). A distance scale 630, in this case in meters, is also provided to allow the user to determine the approximate distance travelled on a particular wave. For example, the 1^{st} wave bar terminates on/around the 160m mark, indicating approximately 160m was travelled while surfing the first wave. Further, the interface screen provides a visual indication of the wave on which the surfer travelled the furthest during the session (in this case the first wave, which is visually distinguished from the other wave bars by having a different colour or shading - e.g. red as opposed to blue). Interface screen also provides a visual indication 632 of the top speed reached during the surfing session (in this case the top speed of 32 Km/h was reached while on the fourth wave as indicated by the lightning bolt icon). Interface screen 620 also displays the following information related to the surf session:
- Wave information 634, in this case indicating the direction of the waves (SE) and the height (2ft).
- Wind information 636, in this case indicating the direction of the wind (NNW) and wind speed (7 knots).
- Tide information 638, in this case indicating a mid-tide.

Interface screen 620 also includes a edit control 640 which can be activated by the user to edit information regarding the surf session (for example, to rate the surf session or override certain of the information related to the surf session).

Interface screen 620 also includes the user's rating 642 of the surf session, (in this case 2.5 stars out of 5), an image 644 associated with the surf session, a short comment 646 in respect of the surf session 648, and board information regarding the board used in the surf session.

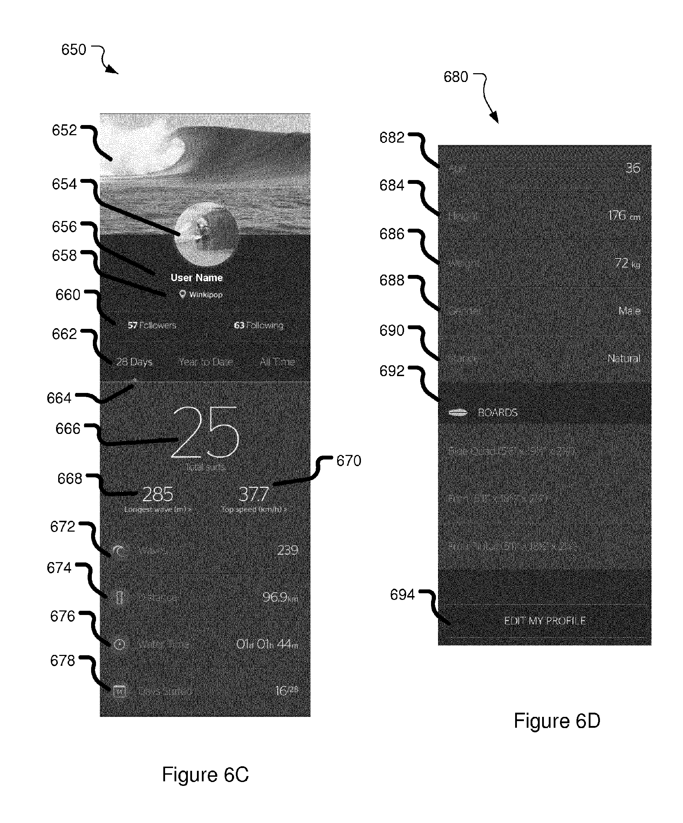

Figure 6C depicts a user interface screen 650 which displays cumulative information regarding the users surf session. In this case interface screen 650 displays:
- A general image/photo 652 provided by the user.
- A profile image 654 provided by the user
- The user's user name 656.
- The user's current location 658.
- Social media information 660, in this case the number of followers of the user (57) and the number of people the user is following (63).
- Viewing period options 662. In this case the user can select to view information over the preceding 28 days, the year to date, or over all time.
- An indicator 664 indicating which viewing period option is currently selected.
- A total number of surfs 666 over the selected viewing period (in this case 25).
- A longest wave 668 over the selected viewing period (in this case 285m).
- A top speed 670 over the selected viewing period (in this case 37.3km/h).
- A total number of waves caught 672 over the selected viewing period (in this case 239 waves).
- A total distance surfed 674 over the selected viewing period (in this case 96.9km).
- A total amount of time in the water 676 over the selected viewing period (in this case 1 day, 1 hour and 44 minutes).
- A total number of days surfed 678 over the selected viewing period (in this case 16 of the 28 days).

Figure 6D depicts a user interface screen 680 which displays user profile information including:
- Age information 682 (in this case 36).
- Height information 684 (in this case 176cm).
- Weight information 686 (in this case 72kg).
- Gender information 688 (in this case male).
- Stance information 690 (in this case natural).
- Board information 692 (in this case a Blue Quad board, a FMN board, and a FMN Pintail board).

Interface screen 680 also displays an edit profile control 694 which, if activated, allows the user to edit various profile information.

In some embodiments the application also provides sharing functionality by which a surfer can to share information concerning his or her surf session(s) with others and/or view information relating to the surfing sessions of other surfers.

For example, a surfer may share information on a session by communicating the relevant data directly to another person's computing device (the other person's computing device having appropriate software and hardware to receive and view the data). In addition, or alternatively, the surfer may share surf session information by posting the relevant data on a website and allowing certain people access to that data (for example in a similar fashion to the Strava website used by runners and cyclists).

The application may also, or alternatively, allow a surfer to post selected information regarding a surf session directly to a social media service. For example, the application may provide a control operable by the user which, when activated, generates an automatic facebook update with certain predefined information (e.g. the location of the surf, the number of waves caught, and the user's rating of the session).

### Alternative embodiments

While the invention has been described in relation to the activity of surfing, the principles of the invention can also be applied to alternative activities/sports.

For example, the principles described above could be applied to other wave-based sports, such as surf skiing and paddle boarding.

The principles above may also be applied to land-based sports where participants move and repeat "runs" within a sporting venue, such as skiing or snowboarding within a snowfield. In this case rather than determining wave start and end points, run start and end points are determined. This can be done in a similar fashion to that described above with speed thresholds and time windows selected as appropriate for the activity in order to determine (for example) periods spent actually skiing/snowboarding versus periods spent riding a chair lift or T-bar. When applying features of the present invention to a run-based activity, and using the speed determination example: an on-run speed threshold is set rather than an on-wave speed threshold; an off-run speed threshold is set rather than an off-wave speed threshold; an on-run time window is set rather than an on-wave time window; and an off-run time window is set rather than an off-wave time window.

It will be understood that the invention disclosed and defined in this specification extends to all alternative combinations of two or more of the individual features mentioned or evident from the text or drawings. All of these different combinations constitute various alternative aspects of the invention.

## Claims

1. A computer processing system including:
a processing unit configured to:
access activity data relating to a user's movements during a session;
process said activity data to identify one or more wave start points and one or more wave end points; and
estimate one or more waves caught by the user during the session, each wave being defined by a wave start point and a corresponding wave end point.

2. The computer processing system according to claim 1, wherein:
the activity data includes speed data recorded over the course of the session; and
the processing unit is configured to process said speed data to identify one or more wave start and/or wave end points.

3. The computer processing system according to claim 2, wherein identifying a wave start point includes processing the activity data to identify a point in the activity data where speed exceeds a predetermined on-wave speed threshold, preferably for longer than a predetermined on-wave time window.

4. The computer processing system according to any one of claims 1 to 3, wherein:
the activity data includes positional data recorded over the course of the session; and
the processing unit is configured to process said positional data to identify one or more wave start and/or a wave end points.

5. The computer processing system according to claim 4, wherein identifying a wave start point includes processing the activity data to identify a point in the activity data where position deviates by more than a predetermined on-wave angle.

6. The computer processing system according to any one of claims 1 to 5, wherein the processing unit is further configured to calculate a total on-wave time during the session, the total on-wave time being the sum of the durations of the one or more waves caught.

7. The computer processing system according to any one of claims 1 to 6 wherein:
the computer processing system further includes a display;
the activity data includes positional data recorded over the course of the session; and wherein
the processing unit is further configured to display a position of at least one of the waves estimated to have been caught during the session.

8. The computer processing system according to any one of claims 1 to 7, wherein the activity data is recorded by a portable device carried by the user during the session, and wherein the activity data is received by the computer processing system from the portable device.

9. The computer processing system according to any one of claims 1 to 8 further including a position sensor, and wherein:
the activity data includes data sensed by the position sensor; and
the computer processing system is a portable device, for instance a wearable device, configured to be carried by the user during the session.

10. A computer-implemented method for estimating one or more waves caught by a user during a session, the method including:
accessing activity data relating to a user's movements during a session;
operating a computer processing unit to process said activity data to identify one or more wave start points and one or more wave end points; and
estimating one or more waves caught by the user during the session, each wave being defined by a wave start point and a corresponding wave end point.

11. The computer-implemented method according to claim 10, wherein the activity data includes speed data recorded over the course of the session; and wherein the method includes identifying one or more wave start and/or wave end points from the speed data.

12. The computer-implemented method according to claim 11, wherein identifying a wave start point includes identifying a point in the activity data where speed exceeds a predetermined on-wave speed threshold, and/or wherein identifying a wave start point includes identifying a point in the activity data where speed has exceeded a predetermined on-wave speed threshold for longer than a predetermined on-wave time window.

13. The computer processing system according to any one of claims 10 to 12, wherein the activity data includes positional data recorded over the course of the session; and the method includes identifying one or more wave start and/or a wave end points from the positional data, preferably wherein identifying a wave start point includes identifying a point in the activity data where position deviates by more than a predetermined on-wave angle.

14. The computer-implemented method according to any one of claims 10 to 13, further including:
calculating a total on-wave time during the session, the total on-wave time being the sum of the durations of the one or more waves caught; and/or displaying a position of at least one of the waves estimated to have been caught during the session on a display.

15. A non-transient computer readable medium storing instructions executable by a computer processing unit to cause a computer processing system to perform a computer implemented method according to any one of claims 10 to 14.
